(19)

Europäisches
Patentamt
European
Patent Office
Office européen
des brevets

(11) **EP 3 968 337 A1**

(12) **EUROPEAN PATENT APPLICATION**
published in accordance with Art. 153(4) EPC

(43) Date of publication:
**16.03.2022 Bulletin 2022/11**

(21) Application number: **20801439.9**

(22) Date of filing: **22.04.2020**

(51) International Patent Classification (IPC):
***G16H 50/20*** (2018.01)

(52) Cooperative Patent Classification (CPC):
**G16H 50/20; G16H 50/70**

(86) International application number:
**PCT/CN2020/086007**

(87) International publication number:
**WO 2020/224433 (12.11.2020 Gazette 2020/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.05.2019 CN 201910386448**

(71) Applicant: **TENCENT TECHNOLOGY (SHENZHEN) COMPANY LIMITED**
**Shenzhen, GUANGDONG 518057, (CN)**

(72) Inventors:
• **QIAO, Zhi**
**Shenzhen, Guangdong 518057 (CN)**

• **GE, Shen**
**Shenzhen, Guangdong 518057 (CN)**
• **YAN, Yangtian**
**Shenzhen, Guangdong 518057 (CN)**
• **WANG, Kai**
**Shenzhen, Guangdong 518057 (CN)**
• **WU, Xian**
**Shenzhen, Guangdong 518057 (CN)**
• **FAN, Wei**
**Shenzhen, Guangdong 518057 (CN)**

(74) Representative: **EP&C**
**P.O. Box 3241**
**2280 GE Rijswijk (NL)**

(54) **TARGET OBJECT ATTRIBUTE PREDICTION METHOD BASED ON MACHINE LEARNING AND RELATED DEVICE**

(57) Provided are a target object attribute prediction method based on machine learning and a related device, which belong to the technical field of data prediction. The method determines the global feature of the target object based on the regular feature that represents the history and future change regular of a detection feature, and refines the global feature to obtain at least one local feature of the target object, then the refined local feature can better reflect the characteristics of the target object, further predict the attribute of the target object based on the local feature, which can improve the accuracy of the predicted attribute, when the attribute of the target object is the predicted diagnosis result, the accuracy of the predicted diagnosis result can be improved.

A computer device determines a detection feature of the target object according to detection data of the target object and an attribute corresponding to the detection data — 201

The computer device inputs the detection feature into a first neural network, and for a detection feature in each time series in the detection feature, the first neural network outputs a first rule feature and a second rule feature different from the first rule feature through two different time series calculations — 202

The computer device determines a global feature of the target object based on the first rule feature and the second rule feature — 203

The computer device inputs the global feature into a second neural network, and the second neural network extracts one or more local features of the target object from the global feature and outputs the one or more local features — 204

The computer device predicts an attribute of the target object based on the one or more local features of the target object — 205

FIG. 2

EP 3 968 337 A1

## Description

This application claims priority to Chinese Patent Application 201910386448.4, entitled "ATTRIBUTE PREDICTION METHOD AND APPARATUS, COMPUTER DEVICE, AND COMPUTER-READABLE STORAGE MEDIUM" and filed with the China National Intellectual Property Administration on May 9, 2019, which is incorporated herein by reference in its entirety.

## FIELD OF THE TECHNOLOGY

The present disclosure relates to the technical field of data prediction, and in particular, to prediction for an attribute of a target object based on machine learning.

## BACKGROUND OF THE DISCLOSURE

Electronic health records (EHR) data may record every consultation record of a target object. With the development of technology, more and more clinical diagnosis estimation models may simulate a diagnosis process of a doctor based on EHR data of a patient, so as to predict a future morbidity of a user.

In the related art, the future morbidity of the user may be predicted by performing the following processes. The medical number data in the EHR data, as an attribute of the patient, is inputted into the clinical diagnosis estimation model. The clinical diagnosis estimation model trains the medical number data and outputs a predicted diagnosis result. The process of training the medical number data by the clinical diagnosis estimation model may be considered as a process of the clinical diagnosis estimation model simulating the diagnosis of the doctor, so that the future morbidity of the patient may be predicted subsequently according to the diagnosis result predicted by the clinical diagnosis estimation model.

However, due to a low accuracy of the diagnosis result predicted by the clinical diagnosis estimation model in the diagnosis prediction process, the future morbidity of the patient determined based on the predicted diagnosis result is inaccurate.

## SUMMARY

There are provided a method for predicting an attribute of a target object based on machine learning and a related device according to embodiments of the present disclosure, to resolve a problem of a low accuracy of a diagnosis result predicted by a clinical diagnosis estimation model. The following technical solutions are provided.

According to an aspect, a method for predicting an attribute of a target object based on machine learning is provided. The method is performed by a computer device and includes:

determining a detection feature of the target object according to detection data of the target object and an attribute corresponding to the detection data;

inputting the detection feature into a first neural network;

for a detection feature in each time series in the detection feature, outputting a first rule feature and a second rule feature different from the first rule feature through two different time series calculations by using the first neural network, where the first rule feature indicates a historical change rule of the detection feature, and the second rule feature indicates a future change rule of the detection feature;

determining a global feature of the target object based on the first rule feature and the second rule feature;

inputting the global feature into a second neural network;

extracting one or more local features of the target object from the global feature and outputting the one or more local features by using the second neural network; and

predicting the attribute of the target object based on the one or more local features of the target object.

According to another aspect, an apparatus for predicting an attribute of a target object based on machine learning is provided. The apparatus includes an acquisition module, a calculation module, an extraction module and a prediction module, where

the acquisition module is configured to determine a detection feature of the target object according to detection data of the target object and an attribute corresponding to the detection data;

the calculation module is configured to: input the detection feature into a first neural network; and for a detection feature in each time series in the detection feature, output a first rule feature and a second rule feature different from the first rule feature through two different time series calculations by using the first neural network, where the first rule feature indicates a historical change rule of the detection feature, and the second rule feature indicates a future change rule of the detection feature;

the acquisition module is further configured to determine a global feature of the target object based on the first rule feature and the second rule feature;

the extraction module is configured to input the global feature into a second neural network; and extract one or more local features of the target object from the global feature and output the one or more local features by using the second neural network; and

the prediction module is configured to predict the attribute of the target object based on the one or more local features of the target object.

According to another aspect, a computer device is provided. The computer device includes: a processor and a memory configured to store a computer program. The processor is configured to perform the computer program stored in the memory to implement the operations performed by the foregoing method for predicting an attribute of a target object based on machine learning.

According to another aspect, a computer-readable storage medium is provided. The computer-readable storage medium stores a computer program. The computer program, when executed by a computer, implements the operations performed by the foregoing method for predicting an attribute of a target object based on machine learning.

According to another aspect, a computer program product including instructions is provided. The instructions, when run on a computer, cause the computer to perform the operations performed by the foregoing method for predicting an attribute of a target object based on machine learning.

The technical solutions provided in the embodiments of the present disclosure have the following beneficial effects.

The global feature of the target object is determined based on the rule feature respectively indicating the historical and future change rules of the detection feature, and the global feature is refined to obtain one or more local features of the target object. The refined local feature can better reflect the feature of the target object, and the attribute of the target object is further predicted based on the local feature. In this way, the precision of the predicted attribute can be improved. In a case that the attribute of the target object is a predicted diagnosis result, the precision of the predicted diagnosis result can be improved.

BRIEF DESCRIPTION OF THE DRAWINGS

To describe the technical solutions of the embodiments of the present disclosure more clearly, the following briefly introduces the accompanying drawings required for describing the embodiments. Apparently, the accompanying drawings in the following description show only some embodiments of the present disclosure, and a person of ordinary skill in the art may still derive other drawings from these accompanying drawings without creative efforts.

FIG. 1 is a schematic diagram of an implementation environment according to an embodiment of the present disclosure;

FIG. 2 is a flowchart of a method for predicting an attribute of a target object based on machine learning according to an embodiment of the present disclosure;

FIG. 3 is a schematic diagram of a diagnosis estimation model according to an embodiment of the present disclosure;

FIG. 4 is a schematic structural diagram of an apparatus for predicting an attribute of a target object based on machine learning according to an embodiment of the present disclosure; and

FIG. 5 is a schematic structural diagram of a computer device according to an embodiment of the present disclosure.

DESCRIPTION OF EMBODIMENTS

To facilitate understanding of a method for predicting an attribute provided in the present disclosure, the related art of attribute prediction and research findings of the inventors are first described below.

In the related art, a future morbidity of a user may be predicted by performing the following processes. Medical number data in EHR data, as an attribute of a patient is inputted into a clinical diagnosis estimation model. The clinical diagnosis estimation model trains the medical number data and outputs a predicted diagnosis result. The process of training the medical number data by the clinical diagnosis estimation model may be considered as a process of the clinical diagnosis estimation model simulating the diagnosis of a doctor, so that the future morbidity of the patient may be predicted subsequently according to the diagnosis result predicted by the trained clinical diagnosis estimation model.

It is founded by the inventors in the research for the related art that, in the foregoing diagnosis prediction process, the medical number data inputted into the clinical diagnosis estimation model includes data on thousands of diseases, and each patient may suffer from only one or several of the diseases, and is unlikely to suffer from various diseases. In this case, useful data in the medical number data is distributed relatively sparsely and discretely in the medical number data. Moreover, the medical number data can only indicate that the patient suffered from the disease, but cannot indicate an overall physical condition of the patient. In this case, the predicted diagnosis result outputted by the clinical diagnosis estimation model after training such medical number data has a relative low accuracy, resulting in an inaccurate future morbidity of the patient determined based on the predicted diagnosis result.

To resolve the technical problems in the related art, there is provided a method for predicting an attribute of a target object based on machine learning in the present disclosure. The method includes: determining a detection feature of the target object according to detection data of the target object and an attribute corresponding to the detection data; inputting the detection feature into a first neural network; for a detection feature in each time series in the detection feature, outputting a first rule feature and a second rule feature different from the first rule feature through two different time series calculations by using the first neural network, where the first rule feature indicates a historical change rule of the detection feature, and the second rule feature indicates a future change rule of the detection feature; determining a global feature of the target object based on the first rule feature and the second rule feature; inputting the global feature into a second neural network; extracting one or more local features of the target object from the global feature and outputting the one or more local features by using the second neural network; and predicting the attribute of the target object based on the one or more local features of the target object.

It can be seen that, in the method for predicting an attribute of a target object based on machine learning provided in the present disclosure, the global feature of the target object is determined based on the rule features respectively indicating the historical and future change rules of the detection feature, and the global feature is refined to obtain one or more local features of the target object. The refined local features can better reflect the feature of the target object, and the attribute of the target object is further predicted based on the local features. In this way, the precision of the predicted attribute can be improved. In a case that the attribute of the target object is a predicted diagnosis result, the precision of the predicted diagnosis result can be improved.

The method for predicting an attribute of a target object based on machine learning provided in the embodiments of the present disclosure is implemented based on a machine learning technology. The Machine learning (ML) technology is an interdisciplinary subject involving multiple fields, such as probability theory, statistics, approximation theory, convex analysis, and algorithm complexity theory. In the machine learning technology, how a computer simulates or implements a human learning behavior is studied to acquire new knowledge or skills, and existing knowledge structures are reorganized, so as to keep improving the performance itself. The machine learning technology is a core of the artificial intelligence technology, is a basic way to make the computer intelligent, and is applied to various fields of the artificial intelligence. The machine learning and deep learning generally include technologies such as an artificial neural network, a belief network, reinforcement learning, transfer learning, inductive learning, and learning from demonstrations.

In addition, the machine learning is an important direction of the artificial intelligence (AI) technology. The artificial intelligence is a theory, a method, a technology and an application system in which a digital computer or a machine controlled by a digital computer is used to simulate, extend, and expand human intelligence, perceive an environment, acquire knowledge, and obtain the best result by using the knowledge. In other words, the artificial intelligence technology is a comprehensive technology of computer science, which aims to understand essence of the intelligence and produce a new intelligent machine that can respond in a manner similar to human intelligence. The artificial intelligence technology is intended to study the design principles and implementation methods of various intelligent machines, to enable the machines to have functions of perception, reasoning, and decision-making.

The artificial intelligence technology is a comprehensive discipline, and relates to a wide range of fields including both hardware-level technologies and software-level technologies. The artificial intelligence foundational technologies generally include technologies such as a sensor, a dedicated artificial intelligence chip, cloud computing, distributed storage, a big data processing technology, an operating/interaction system, and electromechanical integration. The artificial intelligence software technologies mainly include several aspects such as a computer vision technology, a speech processing technology, a natural language processing technology, and machine learning/deep learning.

It can be learned from the above that the method for predicting an attribute of a target object based on machine learning provided in the embodiments of the present disclosure relates to the artificial intelligence technology, and in particular to the machine learning in the artificial intelligence technology.

It is to be understood that the method for predicting an attribute of a target object based on machine learning provided in the embodiments of the present disclosure is applicable to a computer device capable of processing data, such as a terminal device or a server. The terminal device may be a smartphone, a computer, a personal digital assistant (PDA), a tablet computer, or the like. The server may be an application server or a Web server. In the actual deployment, the server may be an independent server or a cluster server.

In a case that the method for predicting an attribute of a target object based on machine learning provided in the embodiments of the present disclosure is performed by the terminal device, the terminal device may directly predict the attribute of the target object according to the detection data of the target object inputted by a user and the attribute corresponding to the detection data, and display a prediction result for the user to view. In a case that the method for predicting an attribute of a target object based on machine learning provided in the embodiments of the present disclosure is performed by the server, the server firstly predicts the attribute of the target object according to the detection data of the target object uploaded by the terminal device and the attribute corresponding to the detection data to obtain a prediction result; and sends the prediction result to the terminal device, so that the terminal device displays the received prediction result, for the user to view.

To facilitate understanding of the technical solution provided in the embodiments of the present disclosure, an application scenario to which the method for predicting an attribute of a target object based on machine learning provided in the embodiments of the present disclosure is applicable is exemplarily described by using an example in which the method for predicting an attribute of a target object based on machine learning provided in the embodiments of the present disclosure is applied to the terminal device.

In a possible application scenario of the method for predicting an attribute of a target object based on machine learning provided in the embodiments of the present disclosure, a terminal device and a user are involved. The terminal device is configured to perform the method for predicting an attribute of a target object based on machine learning provided in the embodiments of the present disclosure, to predict the attribute of the target object and obtain a prediction result for the user to view.

After receiving an attribute prediction instruction triggered by the user, the terminal device may determine a detection feature of the target object according to detection data of the target object and an attribute corresponding to the detection data; input the detection feature into a first neural network; and for a detection feature in each time series in the detection feature, output a first rule feature and a second rule feature different from the first rule feature through two different time series calculations by using the first neural network. The first rule feature indicates a historical change rule of the detection feature, and the second rule feature indicates a future change rule of the detection feature. Next, the terminal device determines a global feature of the target object based on the first rule feature and the second rule feature, inputs the global feature into a second neural network, extracts one or more local features of the target object from the global feature and outputs the one or more local features by using the second neural network, and predicts the attribute of the target object based on the one or more local features of the target object, to obtain a prediction result, so that the terminal device displays the prediction result to the user.

It is to be understood that in actual applications, the method for predicting an attribute of a target object based on machine learning provided in the embodiments of the present disclosure may be applied to a server. Based on this, in another possible application scenario of the method for predicting an attribute of a target object based on machine learning provided in the embodiments of the present disclosure, a server, a terminal device, and a user are involved. After receiving an attribute prediction instruction triggered by the user, the terminal device generates an attribute prediction request in response to the attribute prediction instruction, and sends the attribute prediction request to the server, so that the server may perform the following processes after receiving the attribute prediction request sent by the terminal device. The server determines a detection feature of the target object according to detection data of the target object and an attribute corresponding to the detection data, inputs the detection feature into a first neural network, and for a detection feature in each time series in the detection feature, output a first rule feature and a second rule feature different from the first rule feature through two different time series calculations by using the first neural network. The first rule feature indicates a historical change rule of the detection feature, and the second rule feature indicates a future change rule of the detection feature. Next, the server determines a global feature of the target object based on the first rule feature and the second rule feature, inputs the global feature into a second neural network, extracts one or more local features of the target object from the global feature and outputs the one or more local features by using the second neural network, and predicts the attribute of the target object based on the one or more local features of the target object, to obtain a prediction result, so that the server may feed back the obtained prediction result to the terminal device, and the user may view the prediction result on the terminal device.

It is to be understood that the foregoing application scenarios are only examples. In actual applications, the method for predicting an attribute of a target object based on machine learning provided in the embodiments of the present disclosure may also be applied to another application scenario for attribute prediction. The method for predicting an attribute of a target object based on machine learning provided in the embodiments of the present disclosure is not limited herein.

To make objectives, technical solutions, and advantages of the present disclosure clearer, the following further describes implementations of the present disclosure in detail with reference to the accompanying drawings.

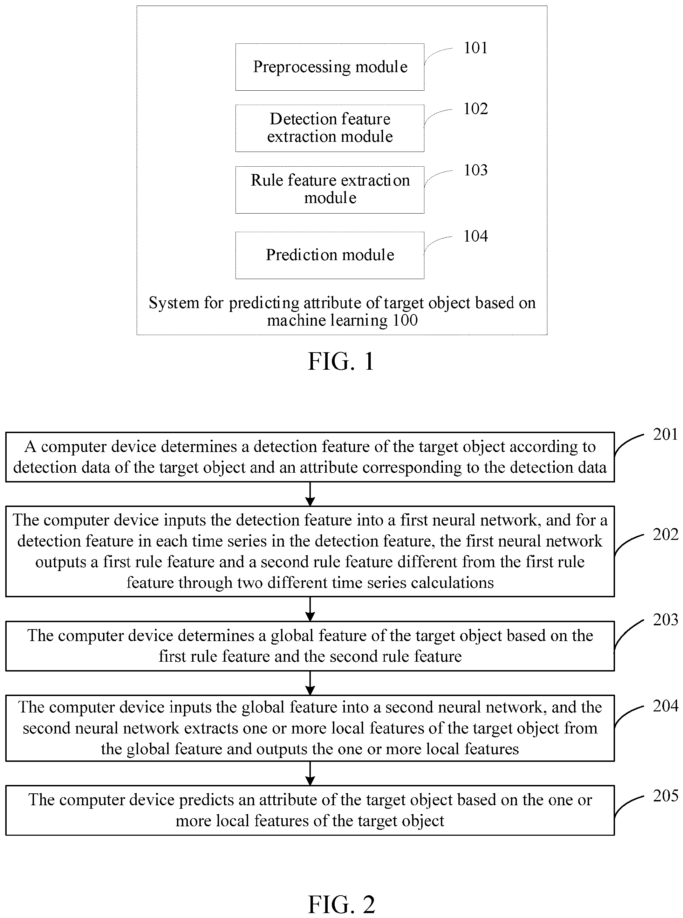

FIG. 1 is a schematic diagram of an implementation environment according to an embodiment of the present disclosure. Referring to FIG. 1, the environment includes a system 100 for predicting an attribute of a target object based on machine learning. The system for predicting an attribute of a target object based on machine learning includes a preprocessing module 101, a detection feature extraction module 102, a rule feature extraction module 103, and a prediction module 104.

The preprocessing module 101 is configured to process detection data of the target object and an attribute corresponding to the detection data. The preprocessing module may transform detection data of a user and an attribute corresponding to the detection data into data that can be processed by the detection feature extraction module 102.

The detection feature extraction module 102 is configured to extract a mixture feature of the feature of the detection data and the attribute corresponding to the detection data. The extracted mixture feature may be used as a detection feature of the target object. The detection feature extraction module 102 may first extract a feature of the attribute and the feature of the detection data based on the data processed by the preprocessing module 101, and splice the feature of the attribute and the feature of the detection data that are extracted. Finally, the detection feature extraction module 102 extracts the detection feature based on a splicing result.

The rule feature extraction module 103 is configured to extract a rule feature and generate a global feature of the target object. The rule feature is used for indicating a global change rule of the detection feature. The rule feature extraction module 103 may firstly extract a historical change rule and a future change rule of the detection feature extracted by the detection feature extraction module 102, and the rule feature extraction module 103 may acquire the global change rule of the detection feature based on the historical change rule and the future change rule of the detection feature, and finally determine the global feature of the target object according to a rule feature indicating the global change rule.

The prediction module 104 is configured to predict the attribute of the target object. The prediction module 104 may refine the global feature generated by the rule feature extraction module 103 by using a neural network, to obtain a local feature of the target object. Next, the prediction module 104 determines a target local feature set to express multiple acquired local features. Finally, the prediction module 104 predicts the attribute of the target object based on the target local feature.

It is to be noted that functions of modules in the system 100 for predicting an attribute of a target object based on machine learning may be implemented by using one computer device or multiple computer devices. A quantity of computer devices that implement the functions of the modules is not limited in the embodiments of the present disclosure.

FIG. 1 shows the functions of the modules in the system for predicting an attribute of a target object based on machine learning. A specific process that the system for predicting an attribute of a target object based on machine learning predicts the attribute is described below. Reference is made to FIG. 2, which is a flowchart of a method for predicting an attribute of a target object based on machine learning according to an embodiment of the present disclosure. As shown in FIG. 2, the method for predicting an attribute of a target object based on machine learning includes the following steps 201 to 205.

In 201, a computer device determines a detection feature of a target object according to detection data of the target object and an attribute corresponding to the detection data.

The computer device may be implemented by any computer device. The target object may be any user.

The detection data of the target object may include detection data of the target object at each detection in a historical time period, and the detection data at each detection corresponds to one detection time. In this case, the detection data at each detection may include multiple types of data related to the target object. Taking physical sign detection for the target object as an example, data detected at each detection may include heartbeat data, blood pressure data, and other types of data. For any type of data, multiple pieces of data may be detected at each detection, and the multiple pieces of data detected at each detection may form a time series sequence related to this detection time. Therefore, one detection time may correspond to multiple time series sequences. Each type of the time series sequence may be labeled to distinguish multiple types of detection data at a same detection time. It is to be noted that the detection time is not limited in this embodiment of the present disclosure, and a time interval between two detection times is not limited herein. In addition, the historical time period is not limited in this embodiment of the present disclosure, and the historical time period may be any time period before the computer device predicts the attribute of the target object by the attribute prediction method.

In the case that the detection data is physical sign data of the target object, the detection data may be time series data stored in the EHR data. The time series data includes an inquiry time of the target object at each inquiry, and physical sign data of the target object detected at each inquiry time. It is to be understood that the inquiry time is also the detection time.

The attribute is used for indicating at least one state of the target object, and each position of the attribute corresponds to one state. A state identifier may be used for indicating whether the target object has a corresponding state. The state identifier may include a first state identifier and a second state identifier. The first state identifier is used for indicating that the target object has the corresponding state, and the second state identifier is used for indicating that the target object does not have the corresponding state. For example, in a case that any one position in the attribute has the first state identifier, it is indicated that the target object has a state corresponding to the position. Further, in a case that any one position in the attribute has the second state identifier, it is indicated that the target object does not have the state corresponding to the position. In addition, different character strings may be used for representing the first state identifier and the second state identifier. The character strings representing the first state identifier or the second state identifier are not limited in this embodiment of the present disclosure.

By detecting the target object each time, current detection data may be obtained. Based on the current detection data, the attribute of the target object may be determined, and the detection time corresponding to the determined attribute of the target object is a detection time corresponding to the current detection data. It can be seen that, in this embodiment of the present disclosure, each attribute corresponds to one detection time. If the detection data of the target object includes at least one detection time, the detection data of the target object corresponds to at least one attribute.

The at least one state may be a sick state of the target object or another state, which is not limited in this embodiment of the present disclosure. In the case that the target object is in the sick state, the attribute corresponding to the detection data may be medical number data. The medical number data may be composed of 0 and 1. Each position in the medical number data corresponds to one disease. In a case that data at any one position is 0, it is indicated that the target object does not suffer from a disease at the position. In a case that the data at any one position is 1, it is indicated that the target object suffers from the disease at the position. It is to be understood that, 0 herein is equivalent to the second state identifier, and 1 herein is equivalent to the first state identifier.

To facilitate calculation, the computer device may firstly preprocess the detection data of the target object and the attribute corresponding to the detection data, so that the detection data and the attribute corresponding to the detection data conform to a format required by subsequent calculation. Next, the computer device performs feature extraction on the processed data to obtain the detection feature of the target object. In a possible implementation, step 201 may be implemented by performing the following steps 2011 to 2014.

In step 2011, the computer device inputs the attribute corresponding to the detection data into a fully connected neural network, screens out a target state in the attribute corresponding to the detection data by using the fully connected neural network, and performs weighting on the target state to output a feature of the attribute corresponding to the detection data.

Because multiple states are stored in the attribute of the target object, the target object has some states in the attribute. The state that the target object has is referred to as the target state.

To facilitate calculation, the computer device preprocesses the attribute corresponding to the detection data. In a possible implementation, the computer device may represent the attribute corresponding to the detection data by using a mulit-hot vector, to preprocess the attribute corresponding to the detection data. The mulit-hot vector is composed of 0 and 1, where 0 indicates that the target object does not have a corresponding state, and 1 indicates that the target object has the corresponding state.

In this case, the computer device may input the mulit-hot vector to the fully connected neural network, and screens out the target state of the target object by the fully connected network using an encoding matrix, and performs weighting on the screened target state to output the feature of the attribute corresponding to the detection data. In the present disclosure, by performing weighting on the screened target state, a processed result can concentrate a feature of the attribute corresponding to the mulit-hot vector.

Each network node in the fully connected network may calculate the data in the mulit-hot vector according to a first formula. The first formula may be expressed as $\pi_j = ReLU(W^T x_j + b_\pi)$ , where $W^T$ represents an encoding feature matrix, which may be a matrix trained in advance, or a matrix trained in a process in which the fully connected neural network calculates the feature of the attribute, $j$ represents a number of a j-th detection time, $x_j$ is a mulit-hot vector of an attribute corresponding to the j-th detection time, that is, $x_j$ represents the mulit-hot vector of the attribute at a j-th detection, $\pi_j$ represents a feature vector of the attribute corresponding to the j-th detection time, the feature vector represents the feature of the attribute at the j-th detection, where j is an integer greater than or equal to 1, and $b_\pi$ represents a bias parameter, which may be a parameter trained in advance, or a parameter trained in the process in which the fully connected neural network calculates the feature of the attribute.

A total quantity of detections of the target object may be represented as J. The computer device calculates all attributes corresponding to the detection data by using the fully connected neural network, and a feature $\pi = [\pi_1,...,\pi_J]$ of the attributes corresponding to the detection data may be obtained. It is to be noted that the computer device may also calculate the feature of the attribute by using a neural network other than the fully connected neural network.

Each attribute is used for indicating at least one state of the target object. In a case that the attribute indicates more states, the mulit-hot vector for representing the attribute has a relatively high dimension. In this case, the dimension of the feature $\pi$ of the attribute is lower than the dimension of the mulit-hot vector by screening the target states, so that the process shown in step 2011 may be regarded as a dimension reduction process, thereby facilitating subsequent calculation.

In step 2012, the computer device inputs the detection data into a time series analysis tool, and extracts a feature of each type of data in the detection data in each time series by using the time series analysis tool to output a feature set.

The time series analysis tool may be implemented by a highly comparative time-series (HCTSA) codebase. The feature of each type of data in each time series may include features respectively representing a data distribution, entropy, and scale attribute and the like of the type of data. It can be learned that the features may indicate an autocorrelation structure of this type of data. Further, since the features are obtained based on actual detection data, the features have interpretability.

After the computer device inputs the detection data at the j-th detection into the time series analysis tool, the time series analysis tool may extract a feature $\left\{S_j^k, k=1,\ldots,z\right\}$ of each type of data from the detection data at the j-th detection based on a preset feature extraction rule, where $S_j^k$ represents a time series feature of a k-th data type at the j-th detection, z is a positive integer, $k$ is a serial number of the data type, j represents a serial number of the j-th detection, and is also a serial number of the j-th detection time, j is a positive integer, $1 \leq j \leq J$, and J represents the total quantity of detections of the target object. It is to be noted that the preset feature extraction rule is not limited in this embodiment of the present disclosure.

J represents the total quantity of detections of the target object. After the detection data of all the J detections is processed by using the time series analysis tool, the time series analysis tool stores the feature of the detection data extracted each time in a feature set $\left\{S_1^k, \ldots, S_J^k, k=1, \ldots, z\right\}$. The time series analysis tool may output the feature set, so that the computer device may obtain the feature set.

Because the features in the feature set can only represent the autocorrelation structure of these types of data, and cannot reflect the feature of the detection data, the computer device also needs to acquire a feature of the detection data by performing the following step 2013.

In step 2013, the computer device inputs the feature set into a deep & cross neural network, and performs cross processing on a feature of each time series in the feature set by using the deep & cross neural network to output a feature of the detection data.

The deep & cross network (DCN) includes a cross network and a deep network. The computer device may input the feature set into the cross network and the deep network. Multiple time series features in the feature set may be crossed by using the cross network, to output the crossed features. Normal features of all the features in the feature set are extracted by using the deep network. The crossed features outputted by the DCN are combined with the normal features extracted by the deep network, to obtain the feature of the detection data.

It is to be noted that the execution sequence of step 2011 to step 203 is not limited in this embodiment of the present disclosure. For example, the computer device may firstly perform step 2011, and then perform step 2012 and step 2013. The computer device may also firstly perform step 2012 and step 2013, and then perform step 2011. The computer device may also perform step 2012 and step 2013 simultaneity when performing step 2011.

In step 2014, the computer device inputs the feature of the attribute and the feature of the detection data into a deep neural network, and extracts a mixture feature of the detection data and the attribute corresponding to the detection data by using the deep neural network, to output the detection feature.

The computer device may firstly splice the feature of the attribute and the feature of the detection data to obtain a spliced feature, and then input the spliced feature into the neural network.

In a possible implementation, the feature $\pi_j$ of the attribute corresponding to the j-th detection and the feature $\tau_j$ of the detection data at the j-th detection are spliced into $\chi_j$ by using a function concat[ ] for connecting arrays, where $\chi_j = concat[\tau_j, \tau_j]$. Then, $\chi_j$ is used as an input of the deep neural network, and each node in the deep neural network may calculate data in $\chi_j$ according to a second formula as follows, so that the deep neural network may output a detection feature $\xi_j$ at the j-th detection time. The second formula may be expressed as $\xi_j = \mathrm{Re}LU\left(W_x^T \chi_j + b_x\right)$, where $W_x$

is a first weight matrix, $W_x^T$ is a transposed matrix of $W_x$, and $b_x$ is a first bias parameter.

Because each weight in $W_x$ is used for representing an importance of each element in $\chi_j$, a weighting process may be performed on each element in $\chi_j$ by using $W_x^T \chi_j$, and the elements in $\chi_j$ may be further integrated. A rectified linear unit (ReLU) function may better mine related features between data. Therefore, the ReLU function has a relatively high expressive ability. $W_x^T \chi_j$ is processed by using the ReLU function, and the result $\xi_j$ obtained by the processing may express the feature included in $\chi_j$. Therefore, $\xi_j$ may be used as the detection feature at the j-th detection.

After a feature of an attribute corresponding to each detection time and a feature of the detection data are spliced, the computer device may extract a detection feature at each detection time by using the deep neural network. For ease of description, the detection feature at each detection time is referred to as a sub detection feature. In this case, the detection feature finally outputted by the deep neural network includes at least one sub detection feature. The computer device may store the at least one sub detection feature in the detection feature according to a time series to obtain a detection feature $\xi = [\xi_1, \xi_2, ... \xi_J]$, where $\xi_j$ is a j-th sub detection feature, i.e., the detection feature corresponding to the j-th detection time, j is a positive integer, $1 \leq j \leq J$, and J represents a total quantity of detection times of the target object.

Because the feature of the detection data and the feature of the attribute include the features of various types of data in each time series, the detection feature has multi-modes, and the detection feature may be regarded as a multi-mode feature.

Because the detection feature is obtained based on the detection data and the attribute corresponding to the detection data, the detection feature in this embodiment of the present disclosure can better reflect the feature of the target object during detection, compared with a case that the feature of the target object is obtained only based on the detection data. Moreover, the detection data is actually detected data, and may be used as an objective basis, so that the obtained detection feature has interpretability. The attribute corresponding to the detection data is a result of a subjective judgment. In this way, the detection feature obtained based on the attribute and the detection data has a relatively high precision.

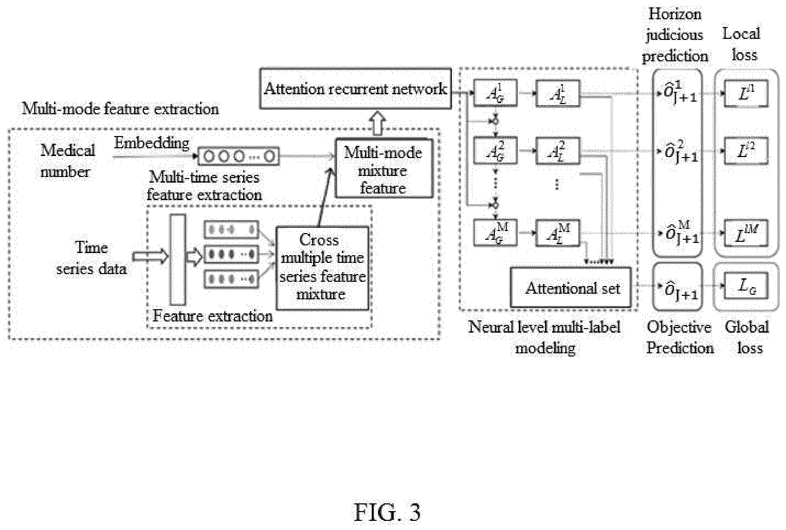

To facilitate understanding of the processes shown in step 2011 to step 2014, the following description is given with reference to a multi-mode feature extraction part shown in FIG. 3, which is a schematic diagram of a diagnosis estimation model according to an embodiment of the present disclosure. In the multi-mode feature extraction part, the computer device firstly converts medical number data (that is, the attribute corresponding to the detection data) into a mulit-hot vector, and inputs the mulit-hot vector into the fully connected neural network. The fully connected neural network may output a feature of the medical number data (that is, the feature of the attribute) through calculation. It is to be noted that the process in which the fully connected neural network outputs the feature of the medical number data through calculation is a process of embedding the medical number data.

Then, the computer device extracts features from time series data (that is, the detection data) to obtain a feature set. Next, the computer device inputs the feature set into the DCN. The DCN outputs cross multiple time series feature mixture, that is, the feature of the detection data. Finally, the computer device mixes the cross multiple time series feature mixture with the feature of the attribute, and acquires the multi-mode feature (that is, the detection feature) based on the feature obtained through mixing.

It is to be noted that the computer device may obtain the feature of the detection data by using a neural network other than the deep neural network.

In 202, the computer device inputs the detection feature into a first neural network, and for a detection feature in each time series in the detection feature, the first neural network outputs a first rule feature and a second rule feature different from the first rule feature through two different time series calculations. The first rule feature indicates a historical change rule of the detection feature, and the second rule feature indicates a future change rule of the detection feature.

The first neural network may be a bidirectional recurrent neural network (BiRNN) with an attention mechanism. The BiRNN may include one first sub-network and one second sub-network. The first sub-network is configured to acquire the first rule feature. The second sub-network is configured to acquire the second rule feature.

In a possible implementation, the computer device inputs the detection feature into the first sub-network of the first neural network according to a backward time series sequence, and performs backward time series calculation on the detection feature by using the first sub-network to obtain the first rule feature. Further, the computer device inputs the detection feature into the second sub-network of the first neural network according to a forward time series sequence, and performs forward time series calculation on the detection feature by using the first sub-network to obtain the second rule feature.

Because the at least one sub detection feature in the detection feature is sorted according to a time sequence, the computer device may input the detection feature into the first sub-network in a forward time series manner, and input the detection feature into the second sub-network in a backward time series manner.

In a possible implementation, the computer device sequentially inputs each sub detection feature in the detection feature $\xi = [\xi_1, \xi_2, \ldots \xi_J]$ into a node of an input layer in the first sub-network according to a sequence from rear to front. Specifically, the computer device inputs $\xi_J$ into a first node of the input layer of the first sub-network, and inputs $\xi_{J-1}$ into a second node of the input layer of the first sub-network, and so on. After the computer device inputs the detection feature into the first sub-network, the first sub-network may calculate each sub-feature in the detection feature based on a preset calculation rule in the first sub-network, so that the first sub-network outputs a first rule feature $h^b = \left[ h_1^b, h_2^b, \ldots h_J^b \right]$, where b is used for representing a backward direction. The preset calculation rule in the first sub-network is not limited in this embodiment of the present disclosure.

In a possible implementation, the computer device sequentially inputs each sub detection feature in the detection feature $\xi = [\xi_1, \xi_2, \ldots \xi_J]$ into a node of an input layer in the second sub-network according to a sequence from front to rear. Specifically, the computer device inputs $\xi_1$ into a first node of the input layer of the first sub-network, and inputs $\xi_2$ into a second node of the input layer of the first sub-network, and so on. After the computer device inputs the detection feature into the second sub-network, the second sub-network may calculate each sub-feature in the detection feature based on a preset calculation rule in the second sub-network, so that the second sub-network outputs a second rule feature $h^f = \left[ h_1^f, h_2^f, \ldots h_J^f \right]$, where f is used for representing a forward direction. The preset calculation rule in the second sub-network is not limited in this embodiment of the present disclosure.

In 203, the computer device determines a global feature of the target object based on the first rule feature and the second rule feature.

Because the historical change rule of the detection feature is indicated by the first rule feature, and the future change rule of the detection feature is indicated by the second rule feature, neither the first rule feature nor the second rule feature can indicate a global change rule of the detection feature. To obtain a more accurate global feature of the target object, the computer device may firstly obtain a rule feature indicating the global change rule of the detection feature based on the first rule feature and the second rule feature, and then obtain the global feature according to the rule feature.

In a possible implementation, step 203 may be implemented through the processes shown in step 2031 to step 2033.

In step 2031, the computer device splices the first rule feature and the second rule feature to obtain a third rule feature.

After the first sub-network outputs the first rule feature $h^b = \left[ h_1^b, h_2^b, \ldots h_J^b \right]$ and the second sub-network outputs the second rule feature $h^f = \left[ h_1^f, h_2^f, \ldots h_J^f \right]$, the computer device may splice the first rule feature by using the first neural network to obtain a third rule feature $h = [h_1, \ldots, h_J]$, where $h_j = [h_j^b, h_j^f]$, j is a positive integer, $1 \leq j \leq J$, and J represents the total quantity of detection times of the target object.

In step 2032, the computer device performs weighting on the third rule feature to obtain a fourth rule feature. The fourth rule feature is used for indicating a global change rule of the detection feature.

The computer device may perform weighting on the third rule feature by using the attention mechanism in the first neural network. In a possible implementation, step 2032 may be implemented through the processes shown in step 11 to step 13.

In step 11, the computer device performs weight learning based on a first attention mechanism and the third rule feature, to obtain one or more first weights. The first weight is used for indicating an importance of one detection data and an attribute corresponding to the one detection data.

The first attention mechanism is any one attention mechanism in the first neural network. The computer device may perform weight learning based on a weight learning policy in the first attention mechanism. The weight learning policy may be a location-based attention weight learning policy, which may be expressed as $e_j^x = W_\tau^T h_j + b_\tau$, where $W_\tau$ is a second weight vector, $W_\tau^T$ is a transposed matrix of $W_\tau$, $b_\tau$ is a second bias parameter, and $e_j^x$ is a first weight corresponding to the j-th detection.

The computer device may perform weight learning based on each rule feature in the third rule feature $h = [h_1, \ldots, h_J]$ and the location-based attention weight learning policy, to obtain J first weights. The J first weights are the one or more first weights.

It is to be noted that the weight learning policy in the first attention mechanism may also be implemented by another attention weight learning policy, which is not limited in this embodiment of the present disclosure.

In step 12, the computer device normalizes the one or more first weights to obtain one or more second weights.

Because the first weight in step 11 is a value obtained through mathematical calculation, the one or more first weights may be excessively large or excessively small. For ease of calculation, the one or more first weights may be normalized, so that each second weight obtained after processing is relatively moderate. If the first weight is excessively large, the first weight may be reduced proportionally. If the first weight is excessively small, the first weight may be enlarged proportionally, to normalize the one or more first weights.

Because each second weight is only a result of normalizing one first weight, the second weight has the same function as the first weight, and both are used for representing an importance of one detection data and an attribute corresponding to the one detection data.

In step 13, the computer device performs weighting on the third rule feature based on the one or more second weights, to obtain a fourth rule feature.

The computer device substitutes the one or more second weights $[\alpha_1^x, \cdots, \alpha_j^x \cdots, \alpha_J^x]$ into a third formula, and uses an output of the third formula as the fourth rule feature to weight the third rule feature. The third formula may be expressed as $$c = \sum_{j=1}^{J} \alpha_j^x h_j$$ , where $c$ is the fourth rule feature, $\alpha_j^x$ is a second weight corresponding to the j-th detection, and J is the total quantity of detection times of the target object.

Because each second weight is used for representing the importance of one detection data and the attribute corresponding to the one detection data, the third rule feature is expressed more intensively by weighting the third rule feature by using at least one second weight. Therefore, the fourth rule feature may represent the global change rule of the detection feature.

Because the first rule feature and the second rule feature are weighted, and the first rule feature and the second rule feature may be integrally represented by the fourth rule feature, so that the fourth rule feature can not only represent the historical change rule expressed by the first rule feature, but also represent the future change rule represented by the first rule feature. Therefore, the fourth rule feature may represent the global change rule of the detection feature.

In step 2033, the computer device determines the global feature based on the third rule feature and the fourth rule feature.

A correlation between adjacent detection data and a correlation between adjacent attributes are the highest. In order to further predict an attribute of the target object during next detection, the computer device may substitute a rule feature corresponding to a last detection time in the third rule feature and the fourth rule feature into a fourth formula, and use an output of the fourth formula as the global feature. The fourth formula may be expressed as

$\hat{c} = ReLU(W_d^T[h_J, c] + b_d)$ , where $\hat{c}$ is the global feature, $h_J$ is the rule feature corresponding to the last detection time of the target object in the third rule feature, $[h_J, c]$ is a vector obtained after $h_J$ and $c$ are spliced, $W_d$ is a third weight matrix, $W_d^T$ is a transposed matrix of $W_d$, $b_d$ is a third bias parameter, and $ReLU( )$ represents a rectified linear unit function.

Because the fourth rule feature indicates the global change rule of the detection feature, the first rule feature may indicate the historical change rule of the detection feature, and the second rule feature may indicate the future change rule of the detection feature, a result obtained by weighting the three rule features can indicate the global feature of the target object.

In 204, the computer device inputs the global feature into a second neural network; and the second neural network extracts one or more local features of the target object from the global feature and outputs the one or more local features of the target object.

The second neural network may be a hierarchical multi-label classification network (HMCN), and the second neural network may extract a local feature from the inputted global feature. Because the global feature cannot represent details of the target object, the details of the target object may be extracted by using the second neural network. The second neural network may extract the details of the target object level by level, so that the finally extracted details can meet requirements of attribute prediction.

Each layer of the second neural network may output one local feature. After the computer device inputs the global feature into the second neural network, the global feature may be inputted from the input layer to an output layer of the second neural network. The second neural network may calculate the global feature layer by layer. A first target layer of the second neural network may calculate a hierarchical feature of the first target layer and a local feature of the target object in the first target layer based on output data of the second target layer, where the first target layer is any one layer of the second neural network, and the second target layer is an upper layer of the first target layer in the second neural network. The hierarchical feature is used for representing a state of the global feature in a network layer of the second neural network, and the hierarchical feature of the first target layer is determined by the global feature and a hierarchical feature of the second target layer.

After the second target layer of the second neural network generates the hierarchical feature of the second target layer and a local feature of the target object in the second target layer, the second target layer may output the hierarchical feature of the second target layer and the global feature (the output data of the second target layer) to the first target layer, so that the first target layer may receive the hierarchical feature of the second target layer and the global feature. As the global feature is outputted by each network layer of the second neural network, the global feature may be inputted to each network layer of the second neural network.

Because the hierarchical feature of the first target layer is determined by the global feature and the hierarchical feature of the second target layer, the first target layer may calculate the hierarchical feature of the first target layer based on the hierarchical feature of the second target layer and the global feature after receiving the hierarchical feature of the second target layer and the global feature. In a possible implementation, a hierarchical feature $A_G^i$ of an i-th layer of the second neural network may be expressed as $A_G^i = \mathrm{Re}\,LU\!\left(W_G^i\!\left(A_G^{i-1} + \hat{c}\right) + b_G\right)$, where $G$ represents global, $W_G^i$ is a fourth weight matrix, $A_G^{i-1}$ is a hierarchical feature of an (i-1)-th layer, $A_G^i$ represents the hierarchical feature of the i-th layer, and $b_G$ is a fourth bias parameter. The i-th layer may be considered as the first target layer.

A node in the first target layer may acquire a local feature of the target object in the first target layer based on the hierarchical feature of the first target layer and the global feature. In a possible implementation, a local feature $A_L^i$ of the target object in the i-th layer may be expressed as $A_L^i = \mathrm{Re}\,LU\!\left(W_T^i A_G^i + b_T\right)$, where $L$ represents a network layer, $W_T^i$ is a fifth weight matrix, and $b_T$ is a fifth bias parameter.

Because each layer of the second neural network performs calculation based on the hierarchical feature of the upper layer and the global feature, the local feature of the target object in each layer of the second neural network is affected by the local feature of the upper layer. Because hierarchical expression of each layer is determined by a hierarchical feature of this layer, a local feature generated by any network layer in the second neural network may function as a parent of a local feature generated by a next network layer. In this way, the second neural network can extract the details of the target object level by level.

In 205, the computer device predicts an attribute of the target object based on the one or more local features of the target object.

One local feature may represent different levels of details of the target object. considering that the quantity of details is large, the details may be processed centrally to obtain a more detailed local feature, and attribute prediction is then performed according to the more detailed local feature.

In a possible implementation, step 205 may be implemented through the processes shown in step 2051 and step 2052.

In step 2051, the computer device performs weighting on the one or more local features of the target object to obtain a target local feature.

The target local feature is also the more detailed local feature. The computer device may perform weighting on the local feature of the target object by using the attention mechanism in the second neural network. In a possible implementation, step 2051 may be implemented through the processes shown in step 21 and step 22.

In step 21, the computer device performs weight learning based on the second attention mechanism and the one or more local features, to obtain one or more third weights. Each third weight is used for representing an importance of one local feature.

The second attention mechanism is any one attention mechanism in the second neural network. The computer device performs weight learning based on the second attention mechanism and the one or more local features, and may learn weights by using a weight learning policy in the second attention mechanism. The weight learning policy in the second attention mechanism may be expressed as:

$$\alpha_i^y = \frac{\exp(e_i^y)}{\sum_{j=1,\dots,M} \exp(e_j^y)}\,, \quad e_i^y = \tanh(W_\alpha A_L^i + b_\alpha)\,, \quad e_i^y \in [e_1^y, \cdots, e_M^y]$$

where $\alpha_i^y$ is an i-th third weight, $W_\alpha$ is a sixth weight matrix, $b_\alpha$ is a sixth bias parameter, $e_j^y$ is a parameter weight corresponding to the j-th detection time, and M represents a quantity of local features.

It is to be noted that the weight learning policy in the second attention mechanism may also be another weight learning policy, which is not limited in this embodiment of the present disclosure.

In step 22, the computer device performs weighting on the one or more local features of the target object based on the one or more third weights, to obtain the target local feature.

The computer device substitutes at least one third weight and at least one local feature of the target object into a fifth formula, and uses an output of the fifth formula as the target local feature to perform weighting on the at least one local feature of the target object. The fifth formula may be expressed as:

$$A_G = \sum_{i=1,\ldots N} \alpha_i A_L^i$$

where $A_G$ is an attribute corresponding to detection data in a current time series, and N is a quantity of layers of the second neural network.

Because each third weight represents an importance of one local feature, the obtained target local feature is more detailed by weighting the one or more local features by using the one or more third weights.

In step 2052, an attribute of the target object is predicted based on the target local feature.

The computer device may substitute the target local feature into a sixth formula to predict the attribute of the target object. The sixth formula is used for predicting the attribute of the target object. The sixth formula may be expressed as $\hat{o}_{J+1} = Sigmoid(W_G A_G + b_G)$, where $\hat{o}_{J+1}$ represents a predicted attribute of the target object corresponding to (J+1)-th data detection,

$W_G$ is a seventh weight matrix, and $b_G$ is a seventh bias parameter.

In some embodiments, the second neural network may determine whether to output a currently predicted attribute according to a global loss and a local loss in the second neural network.

In some possible implementations, after the global feature is inputted to the second neural network, the second neural network outputs the currently predicted attribute if the global loss and the local loss in the second neural network meet a preset condition; otherwise, the second neural network adjusts a weight matrix in the second neural network until the global loss and the local loss in the second neural network meet the preset condition. The local loss is a difference between expected output data and actual output data in each layer of the second neural network. The global loss is a difference between expected final output data and actual final data of the second neural network.

After the calculation of any layer of the second neural network is completed, the second neural network may predict a local feature (referred to as a predicted local feature for short) of the any layer at a next detection time. In this case, a predicted local feature $\hat{o}_{J+1}^i$ of the i-th layer may be expressed as:

$$\hat{o}_{J+1}^i = Sigmoid(W_L^i A_L^i + b_L)$$

where $W_L^i$ is an eighth weight matrix of the i-th layer, and $b_L$ is an eighth bias parameter.

The second neural network may predict an attribute of at least one target object. When predicting the attribute of the at least one target object based on the second neural network, the second neural network may calculate a local loss of any one layer based on a predicted local feature of the any one layer by using a cross entropy policy. In this case, a local loss $L^{li}$ of the i-th layer may be expressed as:

$$L^{li} = -\frac{1}{Q} \sum_{i=1}^{N} o_{J+1}^{(i,Q)} \log\left(\hat{o}_{J+1}^{(i,Q)}\right) + (1 - o_{J+1}^{(i,Q)})\log(1 - \hat{o}_{J+1}^{(i,Q)})$$

where Q is quantity of target objects, $o_{J+1}^{(i,Q)}$ is actual output data of the i-th layer based on a global feature of a Q-th target object, and $\hat{o}_{J+1}^{(i,Q)}$ is predicted output data of the i-th layer based on the global feature of the Q-th target object.

When predicting the attribute of the at least one target object based on the second neural network, if the calculation of each layer of the second neural network is completed, the second neural network may calculate the attribute of the at least one target object during next detection. The second neural network may calculate a global loss $L^G$ based on the predicted attribute of the at least one target object during next detection by using the cross entropy policy. The $L^G$ may be expressed as:

$$L^G = -\frac{1}{Q} \sum_{i=1}^{N} o_{J+1}^{Q} \log(\hat{o}_{J+1}^{Q}) + (1 - o_{J+1}^{Q})\log(1 - \hat{o}_{J+1}^{Q})$$

where $o_{J+1}^{Q}$ is an actually outputted attribute of the Q-th target object during next detection, and $\hat{o}_{J+1}^{Q}$ is a predicted attribute of the Q-th target object during next detection.

The preset condition may be expressed as $Loss = L^G + \gamma(L^{l1} + L^{l2}...L^{lp})$, where P is an integer greater than 2, for example, p = 3, $Loss$ is a preset convergence value, and $\gamma$ is a predefined parameter for balancing the global loss and the local loss. The computer device inputs $Loss$, $\gamma$, $L^{l1}$, $L^{l2}$, ..., and $L^{lp}$ into the formula of the preset condition. If the formula holds, the global loss and the local loss in the second neural network meet the preset condition; otherwise, the global loss and the local loss in the second neural network do not meet the preset condition.

In a case that the global loss and the local loss in the second neural network meet the preset condition, it is indicated that a difference between the generated local feature and the expected local feature of each layer of the second neural network reaches preset precision, thereby ensuring relatively high precision of the local feature of each layer of the second neural network and further improving the precision of the predicted attribute.

It is to be noted that, the second neural network performs calculation based on values, and each state in the attribute of the target object is actually represented by a state identifier, in this case, the computer device further needs to convert the data actually outputted by the second neural network into an attribute formed by the state identifier. The data actually outputted by the second neural network may include at least one probability value. Each probability value corresponds to one state in the attribute of the target object. In a case that any one probability value is greater than a target value, it is indicated that the target object has a target state corresponding to the any one probability, and the computer device stores the first state identifier in a position of the target state in the attribute. In a case that any one probability value is less than or equal to the target value, it is indicated that the target object does not have the target state corresponding to the any one probability, the computer device stores the second state identifier in the position of the target state in the attribute. In this way, an actual expression of the attribute can be obtained by making determination on each probability value. The target value is not limited in this embodiment of the present disclosure.

To further illustrate the processes shown in step 203 and step 204, the following description is given with reference to the neural level multi-label modeling part in FIG. 3. It can be seen from this part that output data (that is, the global feature) of an attention recurrent network is inputted to the neural level multi-label modeling part, and the attention recurrent network is equivalent to the first neural network. The computer device inputs the global feature into each layer of the second neural network in the neural level multi-label modeling part. The first layer generates a hierarchical feature $A_G^l$ of the first layer according to the global feature, and further generates a local feature $A_L^l$ of the first layer according to $A_G^l$. Data prediction may be performed based on $A_L^l$ to obtain output data $\hat{o}_{J+1}^1$ predicted by the first layer. The computer device calculates a local loss $L^{l1}$ of the first layer. The first layer outputs $A_G^l$ to the second layer, so that the second layer may perform a calculation process similar to that of the first layer. Finally, each of all layers of the second neural network may obtain one $A_L^i$. The computer device outputs M $A_L^i$ to an attentional ensembel. In the attention ensemble, predicted output data $\hat{o}_{J+1}$ is generated based on the second attention mechanism. A global loss $L_G$ is generated according to the predicted output data $\hat{o}_{J+1}$. When the global loss $L_G$ and the local losses $L_i$ both meet the present condition, the second neural network may output $\hat{o}_{J+1}$.

In the method provided in the embodiments of the present disclosure, the global feature of the target object is determined based on the rule features respectively indicating the historical and future change rules of the detection feature, and the global feature is refined to obtain one or more local features of the target object. The refined local features can better reflect the feature of the target object, and the attribute of the target object is further predicted based on the local features. In this way, the precision of the predicted attribute can be improved. In a case that the attribute of the target object is a predicted diagnosis result, the precision of the predicted diagnosis result can be improved. In addition, because the detection feature is obtained based on the detection data and the attribute corresponding to the detection data, the detection feature in this embodiment of the present disclosure can better reflect the feature of the target object during detection, compared with the case that the feature of the target object is obtained only based on the detection data. Moreover, the detection data is actually detected data, and may be used as an objective basis, so that the obtained detection feature has interpretability. The attribute corresponding to the detection data is a result of a subjective judgment. In this way, the detection feature obtained based on the attribute and the detection data has a relatively high precision. Furthermore, in the case that the global loss and the local loss in the second neural network meet the preset condition, it is indicated that the local feature generated by each layer of the second neural network reaches an expected value, thereby ensuring relatively high precision of the local feature outputted by the output layer of the second neural network.

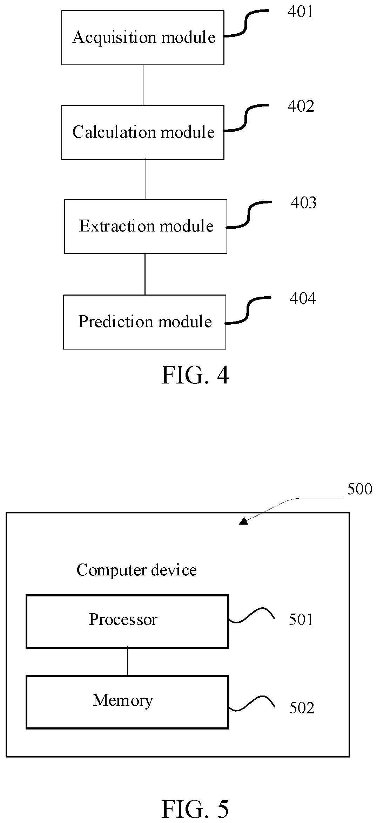

FIG. 4 is a schematic structural diagram of an apparatus for predicting an attribute of a target object based on machine learning according to an embodiment of the present disclosure. The apparatus includes: an acquisition module 401, a calculation module 402, an extraction module 403, and a prediction module 404.

The acquisition module 401 is configured to determine a detection feature of the target object according to detection data of the target object and an attribute corresponding to the detection data.

The calculation module 402 is configured to: input the detection feature into a first neural network; and for a detection feature in each time series in the detection feature, output a first rule feature and a second rule feature different from the first rule feature through two different time series calculations by using the first neural network. The first rule feature indicates a historical change rule of the detection feature. The second rule feature indicates a future change rule of the detection feature.

The acquisition module 401 is further configured to determine a global feature of the target object based on the first rule feature and the second rule feature;

The extraction module 403 is configured to input the global feature into a second neural network; and extract one or more local features of the target object from the global feature and output the one or more local features by the second neural network.

The prediction module 404 is configured to predict the attribute of the target object based on the one or more local features of the target object.

Optionally, the acquisition module 401 is configured to:

input the attribute corresponding to the detection data into a fully connected neural network, delete an unessential factor in the attribute corresponding to the detection data by using the fully connected neural network to obtain a feature of the attribute corresponding to the detection data;

input the detection data into a time series analysis tool, extract a feature of each type of data in the detection data in each time series by using the time series analysis tool to output a feature set;

input the feature set into a deep & cross neural network, and perform cross processing on a feature of each time series in the feature set by using the deep & cross neural network to obtain a feature of the detection data; and

input the feature of the attribute corresponding to the detection data and the feature of the detection data into a deep neural network, and extract a mixture feature of the detection data and the attribute corresponding to the detection data by using the deep neural network to output the detection feature.

Optionally, the acquisition module 401 is configured to:

splice the first rule feature and the second rule feature to obtain a third rule feature;

perform weighting on the third rule feature to obtain a fourth rule feature, the fourth rule feature indicating a global change rule of the detection feature; and

determine the global feature based on the third rule feature and the fourth rule feature.

Optionally, the acquisition module 401 is configured to:

perform weight learning based on a first attention mechanism and the third rule feature, to obtain one or more first weights, where each of the first weights indicates an importance of one detection data and an attribute corresponding to the one detection data;

normalize the one or more first weights to obtain one or more second weights; and

performing weighting on the third rule feature based on the one or more second weights, to obtain the fourth rule feature.

Optionally, the prediction module 404 includes: a processing unit and a prediction unit.

The processing unit is configured to perform weighting on the one or more local features of the target object to obtain a target local feature.

The prediction unit is configured to predict the attribute of the target object based on the target local feature.

Optionally, the processing unit is configured to:

perform weight learning based on a second attention mechanism and the one or more local features, to obtain one or more third weights, where each of the third weights indicates an importance of one of the local features; and

perform weighting on the one or more local features based on the one or more third weights, to obtain the target local feature.

Optionally, each layer of the second neural network outputs one of the local features.

Optionally, the apparatus further includes an output module. The output module is configured to: after the global feature is inputted to the second neural network, output a currently predicted attribute by using the second neural network in a case that a global loss and a local loss in the second neural network meet a preset condition. The local loss is a difference between expected output data and actual output data in each layer of the second neural network. The global loss is a difference between expected final output data and actual final data of the second neural network.

Optionally, the apparatus further includes a generating module. The generating module is configured to generate, based on a hierarchical feature of a first target layer in the second neural network and a local feature generated by a second target layer in the second neural network, a local feature outputted by the first target layer. The hierarchical feature of the first target layer indicates a state of the global feature in the first target layer. The second target layer is an upper layer of the first target layer in the second neural network.

Optionally, the hierarchical feature of the first target layer is determined by the global feature and a hierarchical feature of the second target layer.

FIG. 5 is a schematic structural diagram of a computer device according to an embodiment of the present disclosure. A computer device 500 may vary greatly due to different configurations or performances, and may include one or more central processing units (CPU) 501 and one or more memories 502. The memory 502 stores at least one instruction. The at least one instruction is loaded performed by the processor 501 to implement the method for predicting an attribute of a target object based on machine learning according to the method embodiments. The computer device 500 may further include components such as a wired or wireless network interface, a keyboard, and an input/output interface, to facilitate inputs and outputs. The computer device 500 may further include another component for implementing a function of a device. Details thereof are not described herein.

In an exemplary embodiment, a computer-readable storage medium, for example, a memory including instructions, is further provided. The instructions may be executed by a processor in a terminal to implement the method for predicting an attribute of a target object based on machine learning in the foregoing embodiments. For example, the computer-readable storage medium may be a read-only memory (ROM), a random access memory (RAM), a compact disc read-only memory (CD-ROM), a magnetic tape, a floppy disk, an optical data storage device, or the like.

In an exemplary embodiment, a computer program product including instructions is further provided. The instructions, when run on a computer, cause the computer to perform the method for predicting an attribute of a target object based on machine learning in the foregoing embodiments.

Any combination of the foregoing optional technical solutions may be used to form an optional embodiment of the present disclosure. Details thereof are not described herein.

It is to be noted that when the apparatus for predicting an attribute of a target object based on machine learning according to the foregoing embodiments predicts the attribute, only divisions of the foregoing functional modules are described by using an example. During actual application, the foregoing functions may be allocated to and completed by different functional modules according to requirements, that is, the internal structure of the apparatus is divided into different functional modules, to complete all or some of the foregoing described functions. In addition, the apparatus for predicting an attribute of a target object based on machine learning provided in the foregoing embodiments belongs to the same concept as the embodiments of the method for predicting an attribute of a target object based on machine learning. For the specific implementation process, the method embodiments are referred to, and details thereof are not described herein.

A person of ordinary skill in the art may understand that all or some of the steps of the foregoing embodiments may be implemented by hardware, or may be implemented a program instructing related hardware. The program may be stored in a computer-readable storage medium. The storage medium may be: a read-only memory, a magnetic disk, or an optical disc.

The foregoing descriptions are merely exemplary embodiments of the present disclosure, but are not intended to limit the present disclosure. Any modification, equivalent replacement, or improvement and the like made within the spirit and principle of the present disclosure fall within the protection scope of the present disclosure.

**Claims**

1. A method for predicting an attribute of a target object based on machine learning, performed by a computer device, the method comprising:

   determining a detection feature of the target object according to detection data of the target object and an attribute corresponding to the detection data;
   inputting the detection feature into a first neural network;
   for a detection feature in each time series in the detection feature, outputting a first rule feature and a second rule feature different from the first rule feature through two different time series calculations by using the first neural network, the first rule feature indicating a historical change rule of the detection feature, and the second rule feature indicating a future change rule of the detection feature;
   determining a global feature of the target object based on the first rule feature and the second rule feature;
   inputting the global feature into a second neural network;
   extracting one or more local features of the target object from the global feature and outputting the one or more local features by using the second neural network; and
   predicting the attribute of the target object based on the one or more local features of the target object.

2. The method according to claim 1, wherein the determining a detection feature of the target object according to detection data of the target object and an attribute corresponding to the detection data comprises:

   inputting the attribute corresponding to the detection data into a fully connected neural network, screening out a target state in the attribute corresponding to the detection data by using the fully connected neural network, and performing weighting on the target state to output a feature of the attribute corresponding to the detection data;
   inputting the detection data into a time series analysis tool, and extracting a feature of each type of data in the detection data in each time series by using the time series analysis tool to output a feature set;
   inputting the feature set into a deep & cross neural network, and performing cross processing on a feature of each time series in the feature set by using the deep & cross neural network to obtain a feature of the detection data; and
   inputting the feature of the attribute corresponding to the detection data and the feature of the detection data into a deep neural network, and extracting a mixture feature of the detection data and the attribute corresponding to the detection data by using the deep neural network to output the detection feature.

3. The method according to claim 1, wherein the determining a global feature of the target object based on the first rule feature and the second rule feature comprises:

   splicing the first rule feature and the second rule feature to obtain a third rule feature;
   performing weighting on the third rule feature to obtain a fourth rule feature, the fourth rule feature indicating a global change rule of the detection feature; and
   determining the global feature based on the third rule feature and the fourth rule feature.

4. The method according to claim 3, wherein the performing weighting on the third rule feature to obtain a fourth rule feature comprises:

   performing weight learning based on a first attention mechanism and the third rule feature, to obtain one or more first weights, each of the first weights indicating an importance of one detection data and an attribute corresponding to the one detection data;
   normalizing the one or more first weights to obtain one or more second weights; and
   performing weighting on the third rule feature based on the one or more second weights, to obtain the fourth rule feature.

**5.** The method according to claim 1, wherein the predicting the attribute of the target object based on the one or more local features of the target object comprises:

performing weighting on the one or more local features of the target object to obtain a target local feature; and predicting the attribute of the target object based on the target local feature.

**6.** The method according to claim 5, wherein the performing weighting on the one or more local features of the target object to obtain a target local feature comprises:

performing weight learning based on a second attention mechanism and the one or more local features, to obtain one or more third weights, each of the third weights indicating an importance of one of the local features; and
performing weighting on the one or more local features based on the one or more third weights, to obtain the target local feature.

**7.** The method according to claim 1, wherein each layer of the second neural network outputs one of the local features.

**8.** The method according to claim 1, wherein after inputting the global feature into the second neural network, the method further comprises:
in a case that a global loss and a local loss in the second neural network meet a preset condition, outputting, by the second neural network, a currently predicted attribute, the local loss being a difference between expected output data and actual output data in each layer of the second neural network, and the global loss being a difference between expected final output data and actual final data of the second neural network.

**9.** The method according to claim 1, further comprising:
generating, based on a hierarchical feature of a first target layer in the second neural network and a local feature generated by a second target layer in the second neural network, a local feature outputted by the first target layer, the hierarchical feature of the first target layer indicating a state of the global feature in the first target layer, and the second target layer being an upper layer of the first target layer in the second neural network.

**10.** The method according to claim 9, wherein the hierarchical feature of the first target layer is determined by the global feature and a hierarchical feature of the second target layer.

**11.** An apparatus for predicting an attribute of a target object based on machine learning, the apparatus comprising: an acquisition module, a calculation module, an extraction module and a prediction module, wherein

the acquisition module is configured to determine a detection feature of the target object according to detection data of the target object and an attribute corresponding to the detection data;
the calculation module is configured to: input the detection feature into a first neural network; and for a detection feature in each time series in the detection feature, output a first rule feature and a second rule feature different from the first rule feature through two different time series calculations by using the first neural network, the first rule feature indicating a historical change rule of the detection feature and the second rule feature indicating a future change rule of the detection feature;
the acquisition module is further configured to determine a global feature of the target object based on the first rule feature and the second rule feature;
the extraction module is configured to input the global feature into a second neural network; and extract one or more local features of the target object from the global feature and output the one or more local features by using the second neural network; and
the prediction module is configured to predict the attribute of the target object based on the one or more local features of the target object.

**12.** The apparatus according to claim 11, wherein the acquisition module is configured to:

input the attribute corresponding to the detection data into a fully connected neural network, delete an unessential factor in the attribute corresponding to the detection data by using the fully connected neural network to obtain a feature of the attribute corresponding to the detection data;
input the detection data into a time series analysis tool, extract a feature of each type of data in the detection data in each time series by using the time series analysis tool to output a feature set;

input the feature set into a deep & cross neural network, and perform cross processing on a feature of each time series in the feature set by using the deep & cross neural network to obtain a feature of the detection data; and input the feature of the attribute corresponding to the detection data and the feature of the detection data into a deep neural network, and extract a mixture feature of the detection data and the attribute corresponding to the detection data by using the deep neural network to output the detection feature.

13. The apparatus according to claim 11, wherein the acquisition module is further configured to:

splice the first rule feature and the second rule feature to obtain a third rule feature;
perform weighting on the third rule feature to obtain a fourth rule feature, the fourth rule feature indicating a global change rule of the detection feature; and
determine the global feature based on the third rule feature and the fourth rule feature.

14. A computer device, comprising:

one or more processors; and
one or more memories storing at least one instruction, the at least one instruction being loaded and executed by the one or more processors to implement the method for predicting an attribute of a target object based on machine learning according to any one of claims 1 to 10.

15. A computer-readable storage medium, storing at least one instruction, the at least one instruction being loaded and executed by a processor to implement the method for predicting an attribute of a target object based on machine learning according to any one of claims 1 to 10.

16. A computer program product comprising instructions that, when run on a computer, cause the computer to perform the method for predicting an attribute of a target object based on machine learning according to any one of claims 1 to 10.

```
┌──────────────────────────────────────────────────────┐
│          ┌────────────────────────────┐               │
│          │    Preprocessing module    │  ⌐ 101        │
│          └────────────────────────────┘               │
│          ┌────────────────────────────┐               │
│          │    Detection feature       │  ⌐ 102        │
│          │    extraction module       │               │
│          └────────────────────────────┘               │
│          ┌────────────────────────────┐               │
│          │   Rule feature extraction  │  ⌐ 103        │
│          │          module            │               │
│          └────────────────────────────┘               │
│          ┌────────────────────────────┐               │
│          │     Prediction module      │  ⌐ 104        │
│          └────────────────────────────┘               │
│   System for predicting attribute of target object    │
│            based on machine learning 100              │
└──────────────────────────────────────────────────────┘
```

## FIG. 1

| | |
|---|---|
| A computer device determines a detection feature of the target object according to detection data of the target object and an attribute corresponding to the detection data | ⌐ 201 |
| The computer device inputs the detection feature into a first neural network, and for a detection feature in each time series in the detection feature, the first neural network outputs a first rule feature and a second rule feature different from the first rule feature through two different time series calculations | ⌐ 202 |
| The computer device determines a global feature of the target object based on the first rule feature and the second rule feature | ⌐ 203 |
| The computer device inputs the global feature into a second neural network, and the second neural network extracts one or more local features of the target object from the global feature and outputs the one or more local features | ⌐ 204 |
| The computer device predicts an attribute of the target object based on the one or more local features of the target object | ⌐ 205 |

## FIG. 2

Multi-mode feature extraction

Medical number

Embedding ○○○ ⋯ ○

Multi-time series feature extraction

Time series data

Feature extraction

Cross multiple time series feature mixture

Multi-mode mixture feature

Attention recurrent network

Neural level multi-label modeling

$A_G^1$  $A_L^1$

$A_G^2$  $A_L^2$

$A_G^M$  $A_L^M$

Attentional set

Horizon judicious prediction

$\hat{o}_{J+1}^1$

$\hat{o}_{J+1}^2$

$\hat{o}_{J+1}^M$

Objective Prediction

$\hat{o}_{J+1}$

Local loss

$L^{l1}$

$L^{l2}$

$L^{lM}$

Global loss

$L_G$

FIG. 3

FIG. 4

FIG. 5

# INTERNATIONAL SEARCH REPORT

| International application No. |
|---|
| **PCT/CN2020/086007** |

**A. CLASSIFICATION OF SUBJECT MATTER**

G16H 50/20(2018.01)i

According to International Patent Classification (IPC) or to both national classification and IPC

**B. FIELDS SEARCHED**

Minimum documentation searched (classification system followed by classification symbols)

G16H G06F G06N

Documentation searched other than minimum documentation to the extent that such documents are included in the fields searched

Electronic data base consulted during the international search (name of data base and, where practicable, search terms used)

WPI, EPODOC, CNPAT, CNKI, IEEE: 机器学习, 对象, 属性, 预测, 检测, 特征, 病, 神经网络, 历史, 未来, 全局, 局部, 规律, 双向回归神经网络, machine, learning, object, attribute, prediction, detect, character, feature, disease, neural, network, history, future, global, local, rule, BIRNN

**C. DOCUMENTS CONSIDERED TO BE RELEVANT**

| Category* | Citation of document, with indication, where appropriate, of the relevant passages | Relevant to claim No. |
|---|---|---|
| PX | CN 110111885 A (TENCENT TECHNOLOGY SHENZHEN CO., LTD.) 09 August 2019 (2019-08-09) claims 1-15, description paragraphs 0029-0039, 0169 | 1-16 |
| A | CN 106778014 A (ZHEJIANG UNIVERSITY) 31 May 2017 (2017-05-31) description, paragraphs [0011]-[0025] | 1-16 |
| A | CN 108648829 A (PING AN TECHNOLOGY SHENZHEN CO., LTD.) 12 October 2018 (2018-10-12) entire document | 1-16 |
| A | CN 109698017 A (ZHONGDIAN HEALTH CLOUD TECHNOLOGY CO., LTD.) 30 April 2019 (2019-04-30) entire document | 1-16 |
| A | US 2008010024 A1 (PREDICTION SCIENCES LLP) 10 January 2008 (2008-01-10) entire document | 1-16 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 July 2020** | **22 July 2020** |

| Name and mailing address of the ISA/CN | Authorized officer |
|---|---|
| **China National Intellectual Property Administration (ISA/ CN)** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/086007**

| Patent document cited in search report | | | Publication date (day/month/year) | Patent family member(s) | | | Publication date (day/month/year) |
|---|---|---|---|---|---|---|---|
| CN | 110111885 | A | 09 August 2019 | None | | | |
| CN | 106778014 | A | 31 May 2017 | None | | | |
| CN | 108648829 | A | 12 October 2018 | WO | 2019196286 | A1 | 17 October 2019 |
| CN | 109698017 | A | 30 April 2019 | None | | | |
| US | 2008010024 | A1 | 10 January 2008 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910386448